# EUROPEAN PATENT SPECIFICATION

(11) **EP 4 092 044 B1**
(45) Date of publication and mention of the grant of the patent: **25.09.2024**
(21) Application number: 21741803.7
(22) Date of filing: 14.01.2021
(51) Int. Cl.: C07K 14/47, A61K 38/17, A61P 43/00, A61K 48/00, C12N 15/09

(54) **RECOMBINANT VECTOR COMPRISING CODON-OPTIMIZED TIF1GAMMA POLYNUCLEOTIDE, AND USE THEREOF**
REKOMBINANTER VEKTOR, DER CODON-OPTIMIERTES TIF1GAMMA POLYNUKLEOTID UMFASST, UND VERWENDUNG DAVON
VECTEUR RECOMBINANT COMPRENANT UN POLYNUCLÉOTIDE TIF1GAMMA OPTIMISÉ PAR CODON ET UTILISATION CORRESPONDANTE

(30) Priority: 15.01.2020 KR 20200005239
(43) Date of publication of application: 23.11.2022
(73) Proprietor: KIM, CELL & GENE CO., LTD., Seoul, 04768 (KR)
(72) Inventor: KIM, Hyo-Soo, Seoul 04372 (KR); LEE, Eun Ju, Seoul 02719 (KR)
(74) Representative: Dehns
(86) International application number: PCT/KR2021/000554
(87) International publication number: WO 2021/145704

(56) References cited:
- WO-A2-2013/151665
- KR-A- 20180 011 276
- KR-B1- 101 780 597
- KR-B1- 102 114 880
- US-A1- 2015 218 242
- US-A1- 2020 011 855
- CHEN JIAJING, WANG ZIKANG, GUO XUDONG, LI FUDONG, WEI QINGTAO, CHEN XUWEN, GONG DESHUN, XU YANXIN, CHEN WEN, LIU YONGRUI, KANG JIU: "TRIM 66 reads unmodified H3R2K4 and H3K56ac to respond to DNA damage in embryonic stem cells.", NATURE COMMUNICATIONS, vol. 10, no. 1, 1 December 2019 (2019-12-01), pages 1 - 17, XP055829813, DOI: 10.1038/s41467-019-12126-4
- KULKARNI ATUL, OZA JAY, YAO MING, SOHAIL HONEAH, GINJALA VASUDEVA, TOMAS-LOBA ANTONIA, HOREJSI ZUZANA, TAN ANTOINETTE R., BOULTON : "Tripartite Motif-containing 33 (TRIM33) Protein Functions in the Poly(ADP-ribose) Polymerase (PARP)-dependent DNA Damage Response through Interaction with Amplified in Liver Cancer 1 (ALC1) Protein", JOURNAL OF BIOLOGICAL CHEMISTRY, vol. 288, no. 45, 1 November 2013 (2013-11-01), US, pages 32357 - 32369, XP055829816, ISSN: 0021-9258, DOI: 10.1074/jbc.M113.459164

## Description

### [Background Art]

A fibrotic disease, that is, fibrosis, collectively refers to a disease in which excessive fibrous connective tissue is formed by the abnormal accumulation of collagen matrix in a regeneration or reaction process, and the form thereof is very different from the formation of normal fibrous tissue. Examples of a representative tissue in which fibrosis occurs include the heart, the kidneys, the liver, fat, the lungs, the heart, bones, bone marrow, and the skin. Cardiac fibrosis is a phenomenon commonly occurring in many heart diseases, includes replacement (scarring) fibrosis, interstitial (reactive) fibrosis, and the like, and includes renal fibrosis, hepatic fibrosis, scleroderma, and the like depending on the location of fibrosis, in addition to the fibrosis described above. Scleroderma is a chronic disease characterized by excessive collagen deposition in the skin or other organs and is known to affect blood vessels and internal organs in severe cases. Skeletal muscle fibrosis is a phenomenon that is primarily induced in injured muscles and is characterized by overgrowth of fibrous tissue, primarily caused by the body's attempts to recover from the injury. In particular, recently, pulmonary fibrosis caused by polyhexamethylene guanidine (PHMG) and oligo(2-)ethoxy ethoxyethyl guanidine chloride (PGH) contained in humidifier disinfectants occurred, consequently, causing deaths and becoming a social issue.

Most fibrosis is a disease that is not only highly diverse in pathogenic factors and pathways, but also manifested by repetitive actions, and the mechanism itself has not been clearly identified, so the development of effective drugs for treatment is insufficient (Korean Patent Application Laid-Open No. 10-2019-0003422).

Although examples of an anti-inflammatory agent known to be partially helpful for the treatment of fibrosis include prednisone, glucocorticoids, and the like, the therapeutic effect or stability thereof has not been verified, and there are attempts to use cells differentiated from stem cells with high proliferative capacity instead of tissue transplantation, but it is still difficult to apply this to direct treatment because, *in vivo,* the cell survival rate is low or there is a risk of causing immune rejection. In addition, although methods such as suppression of fibrosis factor activity and extracellular matrix degradation are known for the treatment of fibrosis, not only are the methods still used for direct treatment rare, but also is it difficult to completely treat fibrosis due to the chronic progression of the fibrosis process.

Therefore, there is an urgent need for the development of a substantially commercially viable therapeutic agent that exhibits a remarkable therapeutic effect on various fibrotic diseases.

US2020/011855 discloses a composition for preventing or treating liver fibrosis or cirrhosis, comprising an expression or activity enhancer of TIF1γ as an active ingredient.

WO2013/151665 discloses modified TIF1γ nucleic acids.

### [Disclosure]

### [Technical Problem]

The present invention has been made in an effort to solve the problems in the related art as described above, and an object thereof is to provide a polynucleotide encoding a codon-optimized TIF1γ, a recombinant vector including the same, a use of the recombinant vector, and the like.

However, the technical problems which the present invention intends to solve are not limited to the technical problems which have been mentioned above, and other technical problems which have not been mentioned will be apparently understood by a person with ordinary skill in the art to which the present invention pertains from the following description.

### [Technical Solution]

The present invention provides a polynucleotide, a pharmaceutical composition, a recombinant vector, and a composition for use in preventing or treating a fibrotic disease, as defined in the appended claims.

The present invention provides a polynucleotide in which the N-terminal region of a transcriptional intermediary factor 1 gamma (TIF1γ) gene is codon-optimized, wherein the polynucleotide comprises a nucleic acid sequence of SEQ ID NO: 2. The N-terminus may refer to a gene region corresponding to 359 bp to 1670 bp of the TIP1γ gene, and the codon optimization refers to an optimized sequence in human cells. Preferably, the polynucleotide includes base sequences of SEQ ID NOS: 2 and 3, and may include even more preferably a nucleic acid sequence of SEQ ID NO: 1. Further, the polynucleotide of the present invention includes a variant of SEQ ID NO: 1, 3 or 4 within the scope of the present invention. Specifically, the polynucleotide may include a nucleic acid sequence having a sequence homology of 90 % or more, more preferably 95 % or more, and most preferably 98 % or more to a nucleic acid sequence of SEQ ID NO: 1, 3 or 4. The "% sequence homology" to a polynucleotide is confirmed by comparing a comparison region with an optimally aligned sequence, and a portion of the polynucleotide sequence in the comparison region may further include an addition or deletion (that is, a gap) compared to the reference sequence (without an addition or deletion) for the optimal alignment of the sequence.

In addition, the present invention provides a recombinant vector including the polynucleotide. The recombinant vector may also be used for producing a TIF1γ recombinant protein.

Furthermore, the present invention provides a pharmaceutical composition for preventing or treating a fibrotic disease, including the polynucleotide or recombinant vector.

In addition, the present invention provides a composition for use in preventing or treating a fibrotic disease, including the polynucleotide or recombinant vector as an active ingredient.

In an exemplary embodiment of the present invention, the fibrotic disease is preferably hepatic fibrosis, renal fibrosis, pulmonary fibrosis, pancreatic fibrosis, systemic scleroderma, macular degeneration, cardiac fibrosis, pancreatic and pulmonary cystic fibrosis, injection fibrosis, endomyocardial fibrosis, idiopathic systemic fibrosis, idiopathic pulmonary fibrosis, mediastinal fibrosis, myelofibrosis, retroperitoneal fibrosis, progressive massive fibrosis, nephrogenic systemic fibrosis, nodular subepithelial fibrosis, breast fibrosis, lymph nodal fibrosis, scarring, scleroderma, skin fibrosis, bladder fibrosis, muscle fibrosis, arterial fibrosis, chronic obstructive pulmonary disease, thyroid gland fibrosis, arthrofibrosis, pleural fibrosis, fibrosis as a result of surgery, proliferative fibrosis, pipe-stem fibrosis, postfibrinous fibrosis, and the like, and is more preferably hepatic fibrosis, but is not limited thereto as long as it is a fibrotic disease that can be treated by the expression of the TIP1γ protein.

### [Advantageous Effects]

Since a polynucleotide in which the N-terminal region of the TIF1γ gene is codon-optimized can remarkably increase the production amount of a TIF1γ recombinant protein by suitably modifying the N-terminal region for human cells, it was confirmed that not only the polynucleotide can be used for producing the TIF1γ recombinant protein, but also the therapeutic effect on a fibrotic disease using a recombinant vector into which the polynucleotide is inserted is increased, so the polynucleotide of the present invention is expected to be used as a stable and effective therapeutic agent for the treatment of various fibrotic diseases because the polynucleotide of the present invention can be widely used in various fields where the TIF1γ recombinant protein can be used and can prevent and treat fibrotic diseases by increasing the expression level of the TIP1γ protein.

### [Description of Drawings]

FIG 1 is a view illustrating the results of comparing the protein expression levels of the TIF1γ gene by codon optimization according to an exemplary embodiment of the present invention.
FIG 2 is a view illustrating the results of comparing the protein expression levels of the TIF1γ gene by codon optimization for each region according to an exemplary embodiment of the present invention.
FIG 3 is a view illustrating the results of confirming the therapeutic effect of the codon-optimized recombinant gene of the TIF1γ gene according to an exemplary embodiment of the present invention on fibrotic diseases.
FIG 4 is a view illustrating the results of confirming the therapeutic effect of the codon-optimized recombinant gene of the TIF1γ gene according to an exemplary embodiment of the present invention on fibrotic diseases by H&E staining.
FIG 5 is a view illustrating the results of confirming the therapeutic effect of the codon-optimized recombinant gene of the TIF1γ gene according to an exemplary embodiment of the present invention on fibrotic diseases by MT staining.

### [Modes of the Invention]

Since it was confirmed that when a TIF1γ recombinant protein is produced using a polynucleotide in which the N-terminal region of the TIF1γ gene is codon-optimized, the production amount of the recombinant protein can be increased using the same amount of gene and this also increases the therapeutic effect on fibrotic diseases, it is expected that the optimized TIF1γ polynucleotide of the present invention can be used and applied to various fields such as the treatment of fibrosis.

As used herein, fibrosis collectively refers to all symptoms in which abnormal accumulation of collagen matrix or formation of excessive fibrous connective tissue occurs in a tissue, and a fibrotic disease (fibrosis) may occur due to such fibrosis, and the location of occurrence is not limited. In general, fibrosis is induced when TGF-β1 binds to receptors on the cell wall surface to activate the Smad signaling mechanism, and as a result, the expression levels of α-smooth muscle actin (α-SMA) and a tissue inhibitor of matrix metalloproteinases (TIMP) are increased to activate myofibroblasts and accumulate the matrix, as described in Nature Reviews Nephrology (2016) 12: 325-338.

In the present specification, transcriptional intermediary factor 1 gamma (TIF1γ) is a gene that is known as a transcriptional factor involved in the cell differentiation and development. The TIF1γ of the present invention can treat fibrosis by suppressing Smad4 or forming a complex with Smad2/3 to suppress the expression of genes that induce fibrosis, such as α-SMA. Therefore, the codon-optimized TIF1γ polynucleotide of the present invention can also effectively suppress the expression of genes that induce fibrosis, such as α-SMA, and thus may be applied to various fibrotic diseases such as pulmonary fibrosis, hepatic fibrosis and renal fibrosis.

As used herein, the "vector" refers to a DNA fragment, nucleic acid molecule, and the like, which are delivered into a cell, and the vector may replicate DNA and be independently re-manufactured in a host cell. The vector may be used interchangeably with the term "carrier". "Expression vector" refers to a recombinant DNA molecule that includes a target coding sequence and an appropriate nucleic acid sequence that is essential for expressing an operably linked coding sequence in a specific host organism. The recombinant vector of the present invention includes a plasmid vector, a cosmid vector, a bacteriophage vector, a viral vector, and the like, but is not limited thereto. A suitable expression vector may include an expression regulatory element such as a promoter, an operator, an initiation codon, a termination codon, a polyadenylation signal, and an enhancer, and may be variously manufactured depending on the purpose. The promoter may preferably be a promoter used to express a protein in human cells, and more preferably, a specific promoter inducing expression in a region where fibrosis is induced, such as the TGF-beta promoter, and the like, but is not limited thereto. As used herein, the "operably linked" refers to a state in which a nucleic acid expression control sequence and a nucleic acid sequence encoding a target protein or RNA are functionally linked so as to carry out a general function. For example, a promoter and a nucleic acid sequence encoding a protein or RNA may be operably linked to affect the expression of the coding sequence. An operable linkage with an expression vector may be prepared using a gene recombination technique well-known in the art, and site-specific DNA cleavage and ligation may use enzymes generally known in the art, or the like.

As used herein, prevention refers to a broad concept of blocking the occurrence of fibrosis, preferably includes both primary prevention to prevent the occurrence of fibrosis in advance, and secondary prevention to early detect and timely treat the occurrence, but is not limited thereto as long as it is a process and/or activity which addresses fibrosis before it occurs.

As used herein, treating refers to a broad concept that deals with the occurrence of fibrosis, and is not limited as long as it is a process and/or activity for treating, healing, alleviating, reducing, and the like fibrosis.

As used herein, a subject in need refers to a subject to which the composition of the present invention may be administered, and the subject is not limited.

As used herein, a pharmaceutical composition may be in the form of a capsule, a tablet, a granule, an injection, an ointment, a powder, or a beverage, and the pharmaceutical composition may be characterized in that it targets humans. However, the pharmaceutical composition is not limited thereto and may be formulated into the form of an oral dosage form such as powder, granules, a capsule, a tablet, and an aqueous suspension, an external preparation, a suppository, and a sterile injectable solution. The pharmaceutical composition of the present invention may include a pharmaceutically acceptable carrier. As the pharmaceutically acceptable carrier, a binder, a lubricant, a disintegrant, an excipient, a solubilizing agent, a dispersing agent, a stabilizer, a suspending agent, a colorant, a flavoring agent, and the like may be used when orally administered, in the case of injection, a buffering agent, a preservative, an analgesic, a solubilizer, an isotonic agent, a stabilizer, and the like may be mixed and used, and in the case of topical administration, a base, an excipient, lubricant, a preservative, and the like may be used. The formulation of the pharmaceutical composition of the present invention may be variously prepared by mixing the pharmaceutical composition of the present invention with the pharmaceutically acceptable carrier as described above. For example, the formulation may be prepared in the form of a tablet, a troche, a capsule, an elixir, a suspension, a syrup, a wafer, and the like when orally administered, and in the case of injection, the injection may be formulated into unit dosage ampoules or in multiple dosage forms. The pharmaceutical composition of the present invention may be formulated into other solutions, suspensions, tablets, capsules, sustained-release preparations, and the like.

Meanwhile, as an example of suitable carriers, excipients and diluents for formulation, it is possible to use lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, or the like. Further, the pharmaceutical composition of the present invention may further include a filler, an anticoagulant, a lubricant, a wetting agent, a flavoring agent, an emulsifying agent, an antiseptic, and the like.

The route of administration of the pharmaceutical composition according to the present invention includes, but is not limited to, oral, intravenous, intramuscular, intraarterial, intramedullary, intrathecal, intracardiac, transdermal, subcutaneous, intraperitoneal, intranasal, enteral, topical, sublingual or rectal administration. Oral or parenteral administration is preferred. As used herein, the term "parenteral" includes subcutaneous, intradermal, intravenous, intramuscular, intraarticular, intrasynovial, intrasternal, intrathecal, intralesional administration and intracranial injection or infusion techniques. The pharmaceutical composition of the present invention may also be administered in the form of a suppository for rectal administration.

The pharmaceutical composition of the present invention varies depending on various factors including the activity of the specific compound used, age, body weight, general health, sex, diet, time of administration, route of administration, rate of excretion, drug combination and the severity of the particular disease to be prevented or treated, and the dosage of the pharmaceutical composition varies depending on the condition of the patient, the body weight, the degree of disease, the form of drug, the route of administration and duration, but may be appropriately selected by a person skilled in the art, and may be 0.0001 to 500 mg/kg or 0.001 to 500 mg/kg daily. The administration may be carried out once daily, and may be divided into several times. The dosage is not intended to limit the scope of the present invention in any way. The pharmaceutical composition according to the present invention may be formulated into pills, dragees, capsules, solutions, gels, syrups, slurries, and suspensions.

Hereinafter, preferred examples for helping the understanding of the present invention will be suggested. However, the following examples are provided only to more easily understand the present invention, and the contents of the present invention are not limited by the following examples.

### [Examples]

### Example 1: TIFly codon optimization

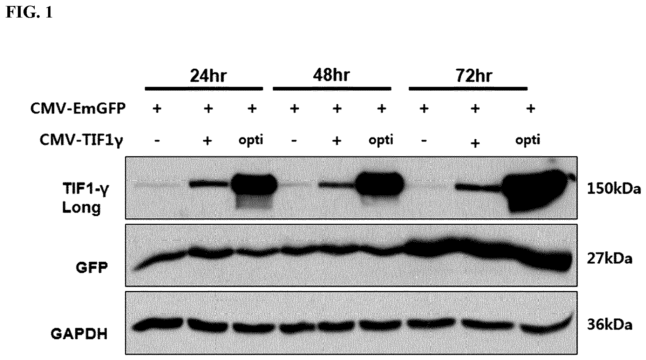

Codon optimization of TIP1γ was performed to maximize the expression of a TIP1γ recombinant protein. In order to confirm the difference in the production amount of recombinant protein according to codon optimization, three types of sequences, in which the entire nucleotide sequence of TIF1γ (Sequence ID: NM 015906) was specifically optimized as gene codons that are frequently expressed in human cells, were prepared. Then, each TIF1γ gene was inserted into pVAX1 (Thermo Fisher) and expressed in a 293T cell line. The original sequence of TIF1γ was used as a control. Further, the efficiency of transfection was compared by transfection with a CMV-emGFP vector. Then, the expressed TIF1γ recombinant protein was confirmed by western blotting. For the western blotting, collected cells were lysed in 0.1% sodium dodecyl sulfate containing a protein lysis buffer (50 mM Tris-HCl, 150 mM NaCl, 0.5% deoxycholate, 1% NP40 and a protease inhibitor cocktail (Roche)), 25 to 30 µg of a protein extract was heated at 95°C for 5 minutes, and then SDS-page was performed. Then, after SDS-page was completed, proteins separated by size were transferred to polyvinylidene fluoride membranes (Millipore) using a BioRad transfer unit (BioRad). Then, the membranes were blocked using 5% skim milk and then reacted using a TIF1γ antibody (Thermo Fisher Scientific), and an anti-GAPDH antibody (ABcam) was used as an internal control. The membrane reacted using the antibodies was washed to remove the unbound antibodies, then reacted using horseradish peroxidase-conjugated secondary antibodies, and bands were confirmed using enhanced chemiluminescence (ThermoFisher). The results are illustrated in FIG 1.

As illustrated in FIG 1, it was confirmed that in the TIF1γ recombinant protein (opti) of SEQ ID NO: 1 among the three codon-optimized sequences, the protein production amount was increased 10-fold or more when compared to the original sequence (+).

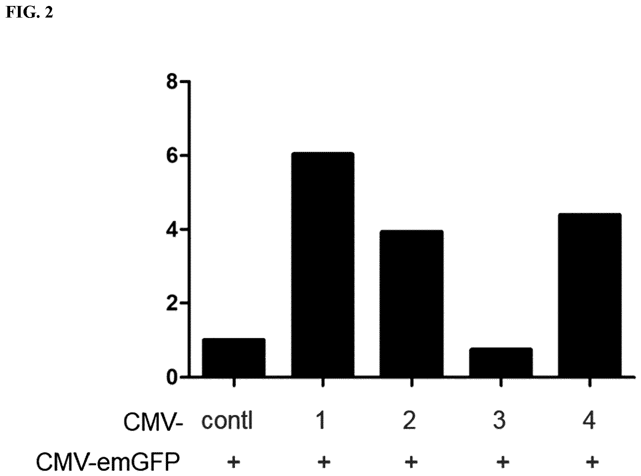

Further, in order to confirm whether the protein expression level varied according to codon optimization of a partial sequence rather than the entire sequence of TIF1γ, based on the codon-optimized sequence of SEQ ID NO: 1, the entire sequence of TIF1γ having a part of the N-terminal portion corresponding to 359 bp to 1670 bp replaced with an optimized sequence (SEQ ID NO: 2), the entire sequence of TIF1γ having a part of the C-terminal portion corresponding to 2560 bp to 3382 bp replaced with an optimized sequence (SEQ ID NO: 3), and the entire sequence of TIF1γ having SEQ ID NOS: 2 and 3 replaced with an optimized sequence (SEQ ID NO: 4) were prepared, inserted into pVAX1, and allowed to be expressed in a 293T cell line for 24 hours. Then, the expressed TIF1γ recombinant protein was confirmed by western blotting, and each band was quantified using ImageJ software. The results are illustrated in FIG 2.

As illustrated in FIG 2, it was confirmed that the protein expression level of the polynucleotide (1) in which the entire sequence of TIF1γ was codon-optimized was the highest, and the polynucleotide (2) in which only the N-terminus was codon-optimized and the polynucleotide (4) in which the N-terminus and C-terminus were codon-optimized had a protein expression level increased 4-fold or more compared to a control in which the original sequence of the TIF1γ gene was used. However, it was confirmed that the polynucleotide (3) in which only the C-terminus was codon-optimized showed almost the same expression level as that of the original sequence. Through the above results, it was confirmed that the codon optimization of the N-terminus plays an important role in the expression level of a TIF1γ recombinant protein.

### Example 2: Confirmation of therapeutic effect of TIFly recombinant protein on hepatic fibrosis disease

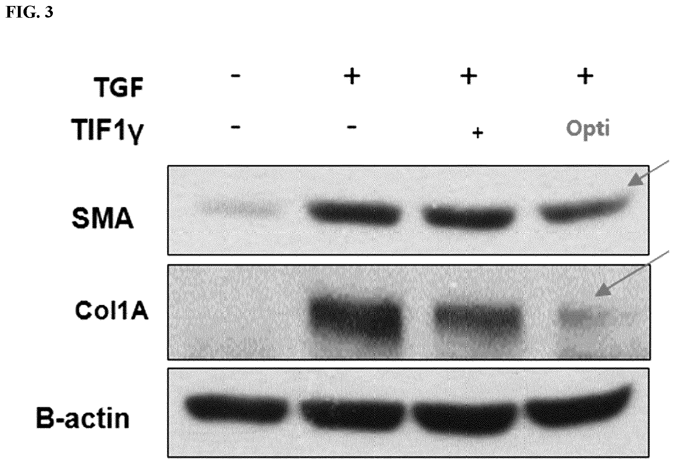

In order to confirm the therapeutic effect of the TIF1γ recombinant protein produced by codon optimization on a hepatic fibrosis disease, a human hepatic stellate cell line LX2 was transfected with a vector constructed in the same manner as in Example 1. Then, the transfected cells were treated with TGFβ at a concentration of 5 ng/mL daily for 7 days. Then, the cells were collected and protein expression levels were confirmed by western blotting. Western blotting was performed in the same manner as in Example 1, and an α-SMA (Abcam) or Col1A (Abcam) antibody was used as a primary antibody. The results are illustrated in FIG 3.

As illustrated in FIG 3, it was confirmed that the expression level of SMA and Col1A, known as fibrotic factors, was increased when cells were treated with TGFβ, the expression level of SMA and Col1A was slightly reduced when cells were transfected with a vector (+) using the original sequence of the TIF1γ gene, and the expression level of SMA and Col1A was further effectively reduced when cells were transfected with a vector (Opti) using a polynucleotide in which the entire sequence of TIF1γ was codon-optimized compared to that of a vector using the original sequence. Through the above results, it was confirmed that the TIF1γ recombinant protein produced by codon optimization showed a fibrosis inhibitory effect as in the case of using the original sequence of the TIF1γ gene, and when cells were transfected with the same amount of vector, the protein expression level in the codon-optimized TIF1γ recombinant increased, and thus the fibrosis therapeutic effect also increased.

### Example 3: Confirmation of therapeutic effect of TIFly recombinant protein on pulmonary fibrosis disease

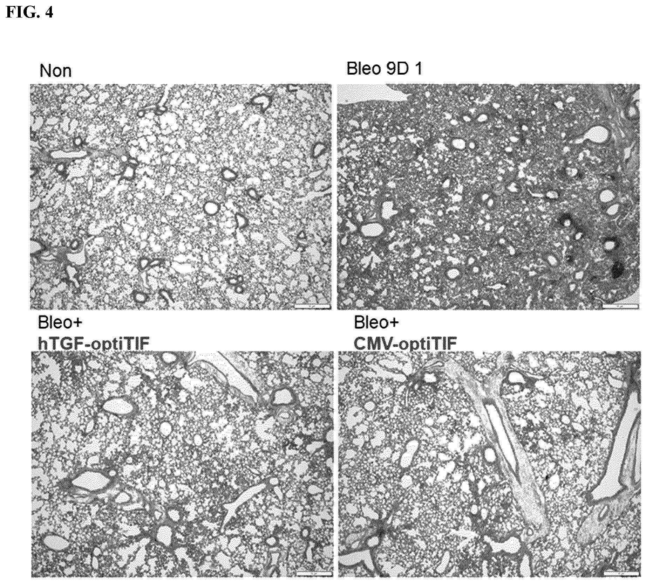

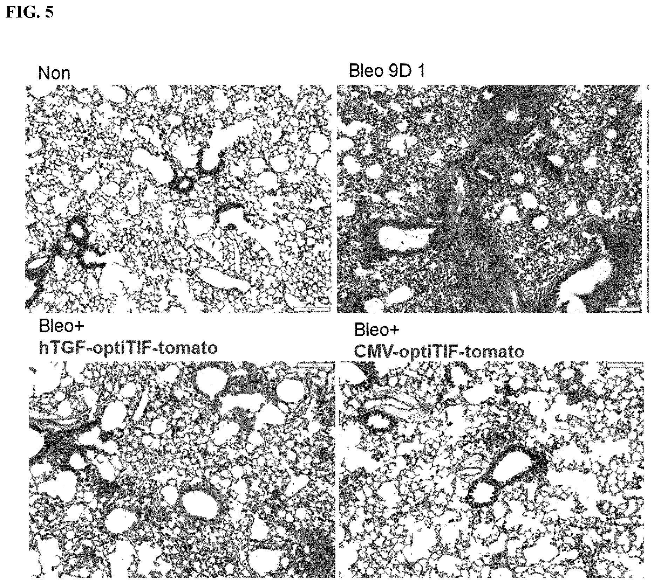

In order to confirm the therapeutic effect of the TIF1γ recombinant protein produced by codon optimization on a pulmonary fibrosis disease, a pulmonary fibrosis animal model was produced by primary treatment with bleomycin. More specifically, bleomycin was injected into 5- to 8-week-old C57BL/6N mouse at a concentration of 2 mg/kg by endotracheal administration, and 9 days later, 18 µg/mouse of a vector constructed in the same manner as in Example 1 was injected via the tail vein. A CMV promoter or a human TGF beta promoter (hTGF) of SEQ ID NO: 5 was used as a promoter sequence of the vector. Then, 21 days later, the mice were euthanized and lung tissue was obtained. The obtained right lung tissue was fixed using a 10% formalin solution, then embedded using paraffin, and cut to a thickness of 4 µm to prepare tissue sections. Then, after the paraffin was removed from the prepared tissue section, immunostaining was performed using hematoxylin and eosin, and then the tissue section was observed using a microscope. The results are illustrated in FIG 4. Then, the obtained left lung tissue was stained using a Masson's trichrome (MT) stain kit (Abcam). The results are illustrated in FIG 5.

As illustrated in FIG 4, it was confirmed that in the lung tissue (Bleo9D1) treated with bleomycin, fibrosis was induced by bleomycin, and when a vector (hTGF-optiTIF or CMV-optiTIF) using a polynucleotide in which the entire sequence of TIF1γ was codon-optimized was injected, fibrosis was reduced.

As illustrated in FIG 5, it was confirmed that in the lung tissue (Bleo9D1) treated with bleomycin, fibrosis was induced by bleomycin, and when a vector (hTGF-optiTIF or CMV-optiTIF) using a polynucleotide in which the entire sequence of TIF1γ was codon-optimized was injected, fibrosis was reduced.

Through the above results, it could be confirmed that fibrosis could be treated using a polynucleotide in which the entire sequence of TIF1γ of the present invention was codon-optimized.

Through the above results, it could be confirmed that when the codon-optimized TIF1γ polynucleotide of the present invention was used, the production amount of the TIF1γ recombinant protein could be increased, and the protein expression level could be increased even though a small amount of vector was injected by using a vector including the codon-optimized TIF1γ polynucleotide, thereby remarkably increasing the therapeutic effect on fibrotic diseases.

### [Industrial Applicability]

Since a polynucleotide in which the N-terminal region of the TIF1γ gene is codon-optimized, wherein the polynucleotide comprises a nucleic acid sequence of SEQ ID NO: 2, and optionally additionally wherein the C-terminal region or entire sequence of the TIF1γ gene according to the present invention is codon-optimized can remarkably increase the production amount of TIF1γ recombinant protein, and it is possible to effectively treat fibrotic diseases using the polynucleotide, the polynucleotide is expected to be widely applied to the treatment of various fibrotic diseases and used as a stable and effective therapeutic agent.

## Claims

1. A polynucleotide in which an N-terminal region of a transcriptional intermediary factor 1 gamma (TIF1γ) gene is codon-optimized, wherein the polynucleotide comprises a nucleic acid sequence of SEQ ID NO: 2.

2. The polynucleotide of claim 1, wherein the polynucleotide comprises a nucleic acid sequence of SEQ ID NO: 1.

3. The polynucleotide of claim 1 or claim 2, wherein the polynucleotide comprises nucleic acid sequences of SEQ ID NOS: 2 and 3.

4. A pharmaceutical composition for preventing or treating a fibrotic disease, comprising the polynucleotide of any one of claims 1 to 3 as an active ingredient.

5. The pharmaceutical composition of claim 4, wherein the fibrotic disease is any one or more selected from the group consisting of hepatic fibrosis, renal fibrosis, pulmonary fibrosis, pancreatic fibrosis, systemic scleroderma, macular degeneration, cardiac fibrosis, pancreatic and pulmonary cystic fibrosis, injection fibrosis, endomyocardial fibrosis, idiopathic systemic fibrosis, idiopathic pulmonary fibrosis, mediastinal fibrosis, myelofibrosis, retroperitoneal fibrosis, progressive massive fibrosis, nephrogenic systemic fibrosis, nodular subepithelial fibrosis, breast fibrosis, lymph nodal fibrosis, scarring, scleroderma, skin fibrosis, bladder fibrosis, muscle fibrosis, arterial fibrosis, chronic obstructive pulmonary disease, thyroid gland fibrosis, arthrofibrosis, pleural fibrosis, fibrosis as a result of surgery, proliferative fibrosis, pipe-stem fibrosis, and postfibrinous fibrosis.

6. A recombinant vector comprising the polynucleotide of any one of claims 1 to 3.

7. A pharmaceutical composition for preventing or treating a fibrotic disease, comprising the recombinant vector of claim 6 as an active ingredient.

8. The pharmaceutical composition of claim 7, wherein the fibrotic disease is any one or more selected from the group consisting of hepatic fibrosis, renal fibrosis, pulmonary fibrosis, pancreatic fibrosis, systemic scleroderma, macular degeneration, cardiac fibrosis, pancreatic and pulmonary cystic fibrosis, injection fibrosis, endomyocardial fibrosis, idiopathic systemic fibrosis, idiopathic pulmonary fibrosis, mediastinal fibrosis, myelofibrosis, retroperitoneal fibrosis, progressive massive fibrosis, nephrogenic systemic fibrosis, nodular subepithelial fibrosis, breast fibrosis, lymph nodal fibrosis, scarring, scleroderma, skin fibrosis, bladder fibrosis, muscle fibrosis, arterial fibrosis, chronic obstructive pulmonary disease, thyroid gland fibrosis, arthrofibrosis, pleural fibrosis, fibrosis as a result of surgery, proliferative fibrosis, pipe-stem fibrosis, and postfibrinous fibrosis.

9. A composition for use in preventing or treating a fibrotic disease, comprising the polynucleotide of any one of claims 1 to 3 as an active ingredient.

10. The composition for use of claim 9, wherein the fibrotic disease is any one or more selected from the group consisting of hepatic fibrosis, renal fibrosis, pulmonary fibrosis, pancreatic fibrosis, systemic scleroderma, macular degeneration, cardiac fibrosis, pancreatic and pulmonary cystic fibrosis, injection fibrosis, endomyocardial fibrosis, idiopathic systemic fibrosis, idiopathic pulmonary fibrosis, mediastinal fibrosis, myelofibrosis, retroperitoneal fibrosis, progressive massive fibrosis, nephrogenic systemic fibrosis, nodular subepithelial fibrosis, breast fibrosis, lymph nodal fibrosis, scarring, scleroderma, skin fibrosis, bladder fibrosis, muscle fibrosis, arterial fibrosis, chronic obstructive pulmonary disease, thyroid gland fibrosis, arthrofibrosis, pleural fibrosis, fibrosis as a result of surgery, proliferative fibrosis, pipe-stem fibrosis, and postfibrinous fibrosis.

11. A composition for use in preventing or treating a fibrotic disease, comprising the recombinant vector of claim 6 as an active ingredient.

12. The composition for use of claim 11, wherein the fibrotic disease is any one or more selected from the group consisting of hepatic fibrosis, renal fibrosis, pulmonary fibrosis, pancreatic fibrosis, systemic scleroderma, macular degeneration, cardiac fibrosis, pancreatic and pulmonary cystic fibrosis, injection fibrosis, endomyocardial fibrosis, idiopathic systemic fibrosis, idiopathic pulmonary fibrosis, mediastinal fibrosis, myelofibrosis, retroperitoneal fibrosis, progressive massive fibrosis, nephrogenic systemic fibrosis, nodular subepithelial fibrosis, breast fibrosis, lymph nodal fibrosis, scarring, scleroderma, skin fibrosis, bladder fibrosis, muscle fibrosis, arterial fibrosis, chronic obstructive pulmonary disease, thyroid gland fibrosis, arthrofibrosis, pleural fibrosis, fibrosis as a result of surgery, proliferative fibrosis, pipe-stem fibrosis, and postfibrinous fibrosis.

## Patentansprüche

1. Polynukleotid, bei dem eine N-terminale Region eines Gens des Transkriptionsintermediärfaktors 1 Gamma (TIF1γ) Codon-optimiert ist, wobei das Polynukleotid eine Nukleinsäuresequenz der SEQ ID NO: 2 umfasst.

2. Polynukleotid nach Anspruch 1, wobei das Polynukleotid eine Nukleinsäuresequenz von SEQ ID NO: 1 umfasst.

3. Polynukleotid nach Anspruch 1 oder Anspruch 2, wobei das Polynukleotid Nukleinsäuresequenzen der SEQ ID NOs: 2 und 3 umfasst.

4. Pharmazeutische Zusammensetzung zur Vorbeugung oder Behandlung einer fibrotischen Erkrankung, die das Polynukleotid nach einem der Ansprüche 1 bis 3 als einen Wirkstoff umfasst.

5. Pharmazeutische Zusammensetzung nach Anspruch 4, wobei die fibrotische Erkrankung eine oder mehrere ist, ausgewählt aus der Gruppe bestehend aus Leberfibrose, Nierenfibrose, Lungenfibrose, Pankreasfibrose, systemischer Sklerodermie, Makuladegeneration, Herzfibrose, pankreatischer und pulmonaler Mukoviszidose, Injektionsfibrose, Endomyokardfibrose, idiopathischer systemischer Fibrose, idiopathischer Lungenfibrose, Mediastinalfibrose, Myelofibrose, retroperitonealer Fibrose, progressiver massiver Fibrose, nephrogener systemischer Fibrose, nodulärer subepithelialer Fibrose, Brustfibrose, Lymphknotenfibrose, Narbenbildung, Sklerodermie, Hautfibrose, Blasenfibrose, Muskelfibrose, Arterienfibrose, chronisch obstruktiver Lungenerkrankung, Schilddrüsenfibrose, Arthrofibrose, Pleurafibrose, Fibrose als Folge einer Operation, proliferativer Fibrose, Röhrenstammfibrose und postfibrinöser Fibrose.

6. Rekombinanter Vektor, der das Polynukleotid nach einem der Ansprüche 1 bis 3 umfasst.

7. Pharmazeutische Zusammensetzung zur Vorbeugung oder Behandlung einer fibrotischen Erkrankung, die den rekombinanten Vektor nach Anspruch 6 als einen Wirkstoff umfasst.

8. Pharmazeutische Zusammensetzung nach Anspruch 7, wobei die fibrotische Erkrankung eine oder mehrere ist, ausgewählt aus der Gruppe bestehend aus Leberfibrose, Nierenfibrose, Lungenfibrose, Pankreasfibrose, systemischer Sklerodermie, Makuladegeneration, Herzfibrose, pankreatischer und pulmonaler Mukoviszidose, Injektionsfibrose, Endomyokardfibrose, idiopathischer systemischer Fibrose, idiopathischer Lungenfibrose, Mediastinalfibrose, Myelofibrose, retroperitonealer Fibrose, progressiver massiver Fibrose, nephrogener systemischer Fibrose, nodulärer subepithelialer Fibrose, Brustfibrose, Lymphknotenfibrose, Narbenbildung, Sklerodermie, Hautfibrose, Blasenfibrose, Muskelfibrose, Arterienfibrose, chronisch obstruktiver Lungenerkrankung, Schilddrüsenfibrose, Arthrofibrose, Pleurafibrose, Fibrose als Folge einer Operation, proliferativer Fibrose, Röhrenstammfibrose und postfibrinöser Fibrose.

9. Zusammensetzung zur Verwendung bei der Vorbeugung oder Behandlung einer fibrotischen Erkrankung, die das Polynukleotid nach einem der Ansprüche 1 bis 3 als einen Wirkstoff umfasst.

10. Zusammensetzung zur Verwendung nach Anspruch 9, wobei die fibrotische Erkrankung eine oder mehrere ist, ausgewählt aus der Gruppe bestehend aus Leberfibrose, Nierenfibrose, Lungenfibrose, Pankreasfibrose, systemischer Sklerodermie, Makuladegeneration, Herzfibrose, pankreatischer und pulmonaler Mukoviszidose, Injektionsfibrose, Endomyokardfibrose, idiopathischer systemischer Fibrose, idiopathischer Lungenfibrose, Mediastinalfibrose, Myelofibrose, retroperitonealer Fibrose, progressiver massiver Fibrose, nephrogener systemischer Fibrose, nodulärer subepithelialer Fibrose, Brustfibrose, Lymphknotenfibrose, Narbenbildung, Sklerodermie, Hautfibrose, Blasenfibrose, Muskelfibrose, Arterienfibrose, chronisch obstruktiver Lungenerkrankung, Schilddrüsenfibrose, Arthrofibrose, Pleurafibrose, Fibrose als Folge einer Operation, proliferativer Fibrose, Röhrenstammfibrose und postfibrinöser Fibrose.

11. Zusammensetzung zur Verwendung bei der Vorbeugung oder Behandlung einer fibrotischen Erkrankung, die den rekombinanten Vektor nach Anspruch 6 als einen Wirkstoff umfasst.

12. Zusammensetzung zur Verwendung nach Anspruch 11, wobei die fibrotische Erkrankung eine oder mehrere ist, ausgewählt aus der Gruppe bestehend aus Leberfibrose, Nierenfibrose, Lungenfibrose, Pankreasfibrose, systemischer Sklerodermie, Makuladegeneration, Herzfibrose, pankreatischer und pulmonaler Mukoviszidose, Injektionsfibrose, Endomyokardfibrose, idiopathischer systemischer Fibrose, idiopathischer Lungenfibrose, Mediastinalfibrose, Myelofibrose, retroperitonealer Fibrose, progressiver massiver Fibrose, nephrogener systemischer Fibrose, nodulärer subepithelialer Fibrose, Brustfibrose, Lymphknotenfibrose, Narbenbildung, Sklerodermie, Hautfibrose, Blasenfibrose, Muskelfibrose, Arterienfibrose, chronisch obstruktiver Lungenerkrankung, Schilddrüsenfibrose, Arthrofibrose, Pleurafibrose, Fibrose als Folge einer Operation, proliferativer Fibrose, Röhrenstammfibrose und postfibrinöser Fibrose.

## Revendications

1. Polynucléotide dans lequel une région N-terminale d'un gène du facteur intermédiaire de transcription 1 gamma (TIF1γ) est optimisée par codon, dans lequel le polynucléotide comprend une séquence d'acide nucléique de SEQ ID NO : 2.

2. Polynucléotide selon la revendication 1, dans lequel le polynucléotide comprend une séquence d'acide nucléique de SEQ ID NO : 1.

3. Polynucléotide selon la revendication 1 ou la revendication 2, dans lequel le polynucléotide comprend des séquences d'acide nucléique de SEQ ID NO : 2 et 3.

4. Composition pharmaceutique pour prévenir ou traiter une maladie fibrotique, comprenant le polynucléotide selon l'une quelconque des revendications 1 à 3 en tant que principe actif.

5. Composition pharmaceutique selon la revendication 4, dans laquelle la maladie fibrotique est l'une ou plusieurs quelconques sélectionnées dans le groupe consistant en la fibrose hépatique, la fibrose rénale, la fibrose pulmonaire, la fibrose pancréatique, la sclérodermie systémique, la dégénérescence maculaire, la fibrose cardiaque, la fibrose kystique pancréatique et pulmonaire, la fibrose au site d'injection, la fibrose endomyocardique, la fibrose systémique idiopathique, la fibrose pulmonaire idiopathique, la fibrose médiastinale, la myélofibrose, la fibrose rétropéritonéale, la fibrose massive progressive, la fibrose systémique néphrogénique, la fibrose sous-épithéliale nodulaire, la fibrose mammaire, la fibrose ganglionnaire lymphatique, les cicatrices, la sclérodermie, la fibrose cutanée, la fibrose vésicale, la fibrose musculaire, la fibrose artérielle, la maladie pulmonaire obstructive chronique, la fibrose de la glande thyroïde, l'arthrofibrose, la fibrose pleurale, la fibrose consécutive à une intervention chirurgicale, la fibrose proliférative, la fibrose en tuyau de pipe et la fibrose postfibrineuse.

6. Vecteur recombinant comprenant le polynucléotide selon l'une quelconque des revendications 1 à 3.

7. Composition pharmaceutique pour prévenir ou traiter une maladie fibrotique, comprenant le vecteur recombinant selon la revendication 6 en tant que principe actif.

8. Composition pharmaceutique selon la revendication 7, dans laquelle la maladie fibrotique est l'une ou plusieurs quelconques sélectionnées dans le groupe consistant en la fibrose hépatique, la fibrose rénale, la fibrose pulmonaire, la fibrose pancréatique, la sclérodermie systémique, la dégénérescence maculaire, la fibrose cardiaque, la fibrose kystique pancréatique et pulmonaire, la fibrose au site d'injection, la fibrose endomyocardique, la fibrose systémique idiopathique, la fibrose pulmonaire idiopathique, la fibrose médiastinale, la myélofibrose, la fibrose rétropéritonéale, la fibrose massive progressive, la fibrose systémique néphrogénique, la fibrose sous-épithéliale nodulaire, la fibrose mammaire, la fibrose ganglionnaire lymphatique, les cicatrices, la sclérodermie, la fibrose cutanée, la fibrose vésicale, la fibrose musculaire, la fibrose artérielle, la maladie pulmonaire obstructive chronique, la fibrose de la glande thyroïde, l'arthrofibrose, la fibrose pleurale, la fibrose consécutive à une intervention chirurgicale, la fibrose proliférative, la fibrose en tuyau de pipe et la fibrose postfibrineuse.

9. Composition pour une utilisation pour prévenir ou traiter une maladie fibrotique, comprenant le polynucléotide selon l'une quelconque des revendications 1 à 3 en tant que principe actif.

10. Composition pour une utiliation selon la revendication 9, dans laquelle la maladie fibrotique est l'une ou plusieurs quelconques sélectionnées dans le groupe consistant en la fibrose hépatique, la fibrose rénale, la fibrose pulmonaire, la fibrose pancréatique, la sclérodermie systémique, la dégénérescence maculaire, la fibrose cardiaque, la fibrose kystique pancréatique et pulmonaire, la fibrose au site d'injection, la fibrose endomyocardique, la fibrose systémique idiopathique, la fibrose pulmonaire idiopathique, la fibrose médiastinale, la myélofibrose, la fibrose rétropéritonéale, la fibrose massive progressive, la fibrose systémique néphrogénique, la fibrose sous-épithéliale nodulaire, la fibrose mammaire, la fibrose ganglionnaire lymphatique, les cicatrices, la sclérodermie, la fibrose cutanée, la fibrose vésicale, la fibrose musculaire, la fibrose artérielle, la maladie pulmonaire obstructive chronique, la fibrose de la glande thyroïde, l'arthrofibrose, la fibrose pleurale, la fibrose consécutive à une intervention chirurgicale, la fibrose proliférative, la fibrose en tuyau de pipe et la fibrose postfibrineuse.

11. Composition pour une utilisation pour prévenir ou traiter une maladie fibrotique, comprenant le vecteur recombinant selon la revendication 6 en tant que principe actif.

12. Composition pour une utilisation selon la revendication 11, dans laquelle la maladie fibrotique est l'une ou plusieurs quelconques sélectionnées dans le groupe consistant en la fibrose hépatique, la fibrose rénale, la fibrose pulmonaire, la fibrose pancréatique, la sclérodermie systémique, la dégénérescence maculaire, la fibrose cardiaque, la fibrose kystique pancréatique et pulmonaire, la fibrose au site d'injection, la fibrose endomyocardique, la fibrose systémique idiopathique, la fibrose pulmonaire idiopathique, la fibrose médiastinale, la myélofibrose, la fibrose rétropéritonéale, la fibrose massive progressive, la fibrose systémique néphrogénique, la fibrose sous-épithéliale nodulaire, la fibrose mammaire, la fibrose ganglionnaire lymphatique, les cicatrices, la sclérodermie, la fibrose cutanée, la fibrose vésicale, la fibrose musculaire, la fibrose artérielle, la maladie pulmonaire obstructive chronique, la fibrose de la glande thyroïde, l'arthrofibrose, la fibrose pleurale, la fibrose consécutive à une intervention chirurgicale, la fibrose proliférative, la fibrose en tuyau de pipe et la fibrose postfibrineuse.
